(19) Europäisches Patentamt
European Patent Office
Office européen des brevets

(11) Veröffentlichungsnummer: **0 084 665**
**B1**

(12) # EUROPÄISCHE PATENTSCHRIFT

(45) Veröffentlichungstag der Patentschrift: **13.08.86**

(21) Anmeldenummer: **82111926.0**

(22) Anmeldetag: **23.12.82**

(51) Int. Cl.⁴: **C 07 D 285/12,** C 07 D 417/12, A 01 N 43/82, A 01 N 47/18, A 01 N 47/36, A 01 N 47/38

(54) **Thiadiazolderivate, Verfahren zu ihrer Herstellung und ihre Verwendung zur Bekämpfung unerwünschten Pflanzenwuchses.**

(30) Priorität: **22.01.82 DE 3201861**

(43) Veröffentlichungstag der Anmeldung: **03.08.83 Patentblatt 83/31**

(45) Bekanntmachung des Hinweises auf die Patenterteilung: **13.08.86 Patentblatt 86/33**

(84) Benannte Vertragsstaaten: **AT BE DE FR GB IT NL**

(56) Entgegenhaltungen:
**EP-A-0 004 071**
**DE-A-2 336 407**
**US-A-4 233 057**
**US-A-4 268 675**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Schirmer, Ulrich, Dr., Berghalde 79, D-6900 Heidelberg (DE)**
Erfinder: **Plath, Peter, Dr., Berner Weg 24, D-6700 Ludwigshafen (DE)**
Erfinder: **Wuerzer, Bruno, Dr. Dipl.- Landwirt, Ruedigerstrasse 13, D-6701 Otterstadt (DE)**

**EP 0 084 665 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft Thiadiazolderivate, Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

Es ist bekannt, daß 3-(5-tert.-Butyl-1,3,4-thiadiazol-2-yl)-1,3-dimethylharnstoff herbizid wirksam ist (BE—SP 765 930) und vor allem zur Unkrautbekämpfung auf Totalflächen verwendet wird. Weiterhin ist bekannt, daß 5-Benzyl-2-amino-thiadiazolderivate herbizid wirksam sind (DE—A—2 336 407) und daß N-(5-Phenoxyethyl-1,3,4-thiadiazol-2-yl)-N'-methyl-N'-(2,2-dimethoxy-ethyl)-harnstoffe u.a. auch N-(5-(1-(2,4-Dichlorphenoxy)ethyl)-1,3,4-thiadiazol-2-yl-N'-methyl-N'-(2,2-dimethoxyethyl)harnstoff Vorprodukte für herbizid wirksame Imidazolidinone sind (US—A—4 268 675 und US—A—4 233 057).

Es wurde gefunden, daß Thiadiazolderivate der Formel

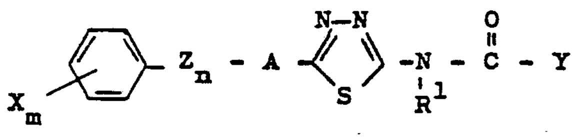

(I),

in der

$R^1$ Wasserstoff oder $C_1$—$C_4$-Alkyl,

A eine gegebenenfalls durch $C_1$—$C_4$-Alkyl substituierte Alkylenkette mit 1 bis 8 Kohlenstoffatomen,

X Wasserstoff, Halogen, Cyano, Nitro, $C_1$—$C_4$-Alkyl, $C_1$—$C_4$-Alkoxy, $C_1$—$C_4$-Halogenalkyl, $C_1$—$C_4$-Halogenalkoxy, $C_1$—$C_4$-Alkylthio, $C_1$—$C_4$-Alkylsulfonyl, $C_3$—$C_6$-Cycloalkyl, Phenyl und gegebenenfalls durch Halogen substituiertes Phenoxy,

m 0, 1, 2, 3 oder 4,

Y $C_1$—$C_4$-Alkyl, $C_3$—$C_6$-Cycloalkyl, $C_1$—$C_4$-Alkoxy, $C_1$—$C_4$-Alkylthio oder den Rest $NR^2R^3$, wobei $R^2$ und $R^3$ unabhängig voneinander Wasserstoff, $C_1$—$C_4$-Alkyl, $C_2$—$C_4$-Alkenyl, $C_2$—$C_4$-Alkinyl, $C_1$—$C_4$-Alkoxy oder $C_3$—$C_6$-Cycloalkyl bedeuten oder $R^2$ mit $R^3$ zusammen eine gegebenenfalls durch Methyl substituierte und eine gegebenenfalls durch Sauerstoff unterbrochene Alkylenkette mit 3 bis 7 Kohlenstoffatomen,

Z Sauerstoff oder Schwefel und

n 0 oder 1 bedeuten und in der anstelle des Restes

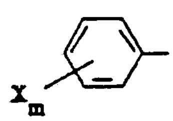

ein gegebenenfalls durch Halogen, $C_1$—$C_4$-Alkyl oder $C_1$—$C_4$-Alkoxy substituierter Naphthylrest stehen kann, eine gute herbizide Wirkung bei zahlreichen wichtigen unerwünschten Pflanzen und eine gute Verträglichkeit bei verschiedenen Kulturpflanzen zeigen.

Die Substituenten in Formel I können die folgenden Bedeutungen haben:

$R^1$ bedeutet Wasserstoff oder $C_1$—$C_4$-Alkyl, wie Methyl, Ethyl, i-Propyl, t.-Butyl,

A bedeutet eine gegebenenfalls durch $C_1$—$C_4$-Alkyl substituierte Alkylenkette mit 1 bis 8 Kohlenstoffatomen, z.B. Methylen, Methylmethylen, Ethylen, Methylethylen, Dimethylmethylen, Dimethylethylen, Propylen, Methylpropylen, Ethylmethylen, Butylen, Pentylen, Hexylen, Heptylen, Methylbutylen, Octylen,

X bedeutet Wasserstoff, Halogen, wie Chlor, Brom, Fluor, Jod, Cyano, Nitro, $C_1$—$C_4$-Alkyl, $C_1$—$C_4$-Alkoxy, $C_1$—$C_4$-Alkylthio, $C_1$—$C_4$-Halogenalkyl, $C_1$—$C_4$-Halogenalkoxy, $C_1$—$C_4$-Alkylsulfonyl, $C_3$—$C_6$-Cycloalkyl, Phenyl oder gegebenenfalls durch Halogen substituiertes Aryloxy, wie gegebenenfalls durch Halogen substituiertes Phenoxy, beispielsweise Methyl, Ethyl, t-Butyl, Methoxy, Ethoxy, t-Butoxy, Methylthio, Ethylthio, n-Butylthio, Trifluoromethyl, Difluormethoxy, 1,1,2-Trifluor-2-chlorethoxy, Methylsulfonyl, Cyclopentyl, Cyclohexyl, Phenoxy, 4-Chlorphenoxy, 2,4-Dichlorphenoxy,

Y bedeutet $C_1$—$C_4$-Alkyl, wie Methyl, Ethyl, t-Butyl, s-Butyl, $C_3$—$C_6$-Cycloalkyl, wie Cyclopropyl, $C_1$—$C_4$-Alkoxy, wie Methoxy, Ethoxy, Isopropoxy, $C_1$—$C_4$-Alkylthio, wie Methylthio, Ethylthio oder den Rest $NR^2R^3$, wobei $R^2$ und $R^3$ unabhängig voneinander Wasserstoff, $C_1$—$C_4$-Alkyl, $C_2$—$C_4$-Alkenyl, $C_2$—$C_4$-Alkinyl, $C_1$—$C_4$-Alkoxy oder $C_3$—$C_6$-Cycloalkyl, wie Methyl, Ethyl, i-Propyl, n-Butyl, t-Butyl, Allyl, Propargyl, 1-Methyl-prop-2-inyl, Methoxy, Ethoxy, n-Propoxy, Cyclopropyl, Cyclopentyl. $R^2$ und $R^3$ können außerdem zusammen eine gegebenenfalls durch Methyl substituierte und gegebenenfalls durch Sauerstoff unterbrochene Alkylenkette mit 3 bis 7 Kohlenstoffatomen bedeuten, z.B. Propylen, Pentylen, Hexylen, 1,4-Dimethylbutylen, 3-Oxapentylen, 2-Oxapentylen, 2-Oxabutylen und

Z bedeutet Sauerstoff oder Schwefel. Anstelle des Restes

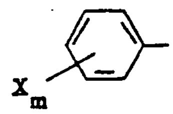

kann in Formel I auch ein gegebenfalls durch Halogen, $C_1$—$C_4$-Alkyl oder $C_1$—$C_4$-Alkoxy substituierter α- oder β-Naphthylrest, wie α-Naphthyl, β-Naphthyl, 4-Chlor-α-naphthyl, 7-Methoxy-α-naphthyl, 2-Methyl-α-naphthyl stehen.

Bevorzugte Thiadiazolderivate der Formel I sind solche, bei denen $R^1$ $C_1$—$C_4$-Alkyl, insbesondere Methyl, und Y den Rest $NR^2R^3$, wobei $R^2$ für Wasserstoff und $R^3$ für $C_1$—$C_4$-Alkyl, isbesondere Methyl, stehen, bedeuten.

Man erhält die Thiadiazolderivate der Formel I durch Umsetzung von Aminothiadiazolen der Formel

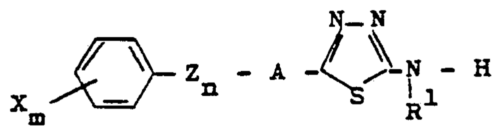 (II),

in der

$R^1$, A, X, Z, m und n die obengenannten Bedeutungen haben, mit Säurechloriden der Formel

Y—CO—Hal (III),

in der Y die obengenannten Bedeutungen hat und Hal Halogen, wie Chlor oder Brom, bedeutet.

Die Umsetzung wird gegebenenfalls in einem inerten organischen Lösungsmittel durchgeführt. Geeignet sind z.B. Kohlenwasserstoffe, wie Ligroin, Benzin, Toluol, Pentan, Cyclohexan, Halogenkohlenwasserstoffe, wie Methylenchlorid, Chloroform, Dichlorethan, Chlorbenzol, o-, m- oder p-Dichlorbenzol, Nitrokohlenwasserstoffe, wie Nitrobenzol, Nitromethan, Nitrile, wie Acetonitril, Butyronitril, Benzonitril, Ether, wie Diethylether, Tetrahydrofuran, Dioxan, Ester, wie Essigsäureethylester, Propionsäuremethylester, Ketone, wie Aceton, Methylethylketon, oder Amide, wie Dimethylformamid, Formamid. Auch Gemische dieser Lösungsmittel können verwendet werden. Die Menge an Lösungsmittel, bezogen auf Aminothiadiazol der Formel II beträgt 100 bis 5000 Gew.%.

Zweckmäßigerweise wird die Umsetzung in Gegenwart eines üblichen Säureakzeptors durchgeführt. In Betracht kommen Alkalihydroxide, Alkalicarbonate, Alkalihydrogencarbonate, Erdalkalihydroxide, Erdalkalicarbonate, Erdalkalihydrogencarbonate, Erdalkalioxide oder Amine, beispielsweise Natriumhydrogencarbonat, Kaliumcarbonat, Triethylamin, Tributylamin, Pyridin, N,N-Dimethylanilin, N,N-Dimethyl-N-cyclohexylamin, Chinolin. Die Menge an Säureakzeptor beträgt 1 bis 4 Mol, bezogen auf 1 Mol Verbindung der Formel III.

Die Ausgangsstoffe der Formeln II und III werden bevorzugt in äquimolarem Verhältnis miteinander umgesetzt. Die Reaktionstemperatur liegt zwischen −20 und +150°C, vorzugsweise zwischen 20 und 80°C.

Außerdem erhält man Thiadiazolderivate der Formel I, in der $R^1$, A, X, Z, m und n die obengenannten Bedeutungen haben und Y den Rest $NR^2R^3$ bedeutet, wobei $R^2$ für Wasserstoff steht und $R^3$ die obengenannten Bedeutungen hat, durch Umsetzung von Aminothiadiazolen der Formel

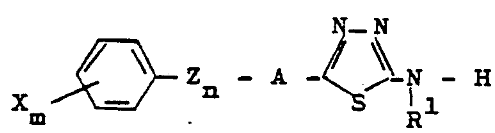 (II),

in der

$R^1$, A, X, Z, m und n die obengenannten Bedeutungen haben, mit Isocyanaten der Formel

$R^3$—NCO (IV),

in der $R^3$ die obengenannten Bedeutungen hat.

Diese Umsetzung wird gegebenenfalls in Gegenwart eines für Isocyanatreaktionen gebräuchlichen Katalysators, z.B. einem tert. Amin, wie Triethylamin, 1,4-Diazabicyclo-[2.2.2.]-octan, stickstoffhaltigen

Heterocyclen, wie Pyridin, 1,2-Dimethylimidazol, oder organischen Zinnverbindungen, wie Dibutylzinndiacetat, Dimethylzinndichlorid und gegebenenfalls in Gegenwart eines unter den Reaktionsbedingungen inerten Lösungsmittels, z.B. eines Kohlenwasserstoffs, wie Ligroin, Benzin, Toluol, Pentan, Cyclohexan, eines Halogenkohlenwasserstoffs, wie Methylenchlorid, Chloroform, Dichlorethan, Chlorbenzol, o-, m- oder p-Dichlorbenzol, eines Nitrokohlenwasserstoffs, wie Nitrobenzol, Nitromethan, eines Nitrils, wie Acetonitril, Butyronitril, Benzonitril, eines Ethers, wie Diethylether, Tetrahydrofuran, Dioxan, eines Esters, wie Essigsäureethylester, Propionsäuremethylester, eines Ketons, wie Aceton, Methylethylketon oder eines Amides, wie Dimethylformamid, Formamid, durchgeführt (Houben-Weyl, Methoden der organ. Chemie, Bd. VIII, S. 132, Georg Thieme-Verlag, Stuttgart, 4. Auflage, 1952). Die Menge an Katalysator beträgt 0,1 bis 5 Mol%, bezogen auf Aminothiadiazol der Formel II; die Lösungsmittelmenge schwankt zwischen 100 und 10 000 Gew.%, bezogen auf Aminothiadiazol der Formel II.

Die Reaktionstemperatur kann zwischen −20 und 150°C, vorzugsweise zwischen 0 und 100°C variieren.

Aminothiadiazole der Formel

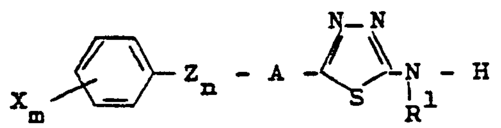 (II),

in der

$R^1$ $C_1$—$C_4$-Alkyl,

A eine gegebenenfalls durch Methyl substituierte Alkylenkette mit 1 bis 8 Kohlenstoffatomen,

X Wasserstoff, Halogen, Cyano, Nitro, $C_1$—$C_4$-Alkyl, $C_1$—$C_4$-Alkoxy, $C_1$—$C_4$-Halogenalkyl, $C_1$—$C_4$-Halogenalkoxy, $C_1$—$C_4$-Alkylthio, $C_1$—$C_4$-Alkylsulfonyl, $C_3$—$C_6$-Cycloalkyl, Phenyl, Aryloxy,

m 0, 1, 2, 3 oder 4,

Z Sauerstoff oder Schwefel und

n 0 oder 1 bedeuten und

in der anstelle des Restes

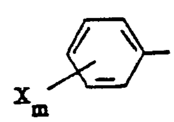

ein gegebenenfalls durch Halogen, $C_1$—$C_4$-Alkyl oder $C_1$—$C_4$-Alkoxy substituierter Naphthylrest stehen kann, erhält man durch Umsetzung von Carbonsäuren der Formel

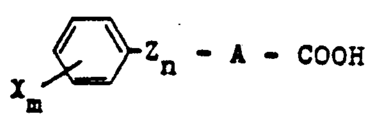 (V),

in der A, X, Z, m und n die obengenannten Bedeutungen haben, mit Thiosemicarbaziden der Formel

$$H_2N—NH—CS—NHR^1 \quad (VI),$$

in der $R^1$ die obengenannten Bedeutungen hat.

Diese Reaktion kann in Gegenwart eines unter den Reaktionsbedingungen inerten Lösungsmitttels durchgeführt werden. Geeignet sind Kohlenwasserstoffe, wie Benzin, Toluol, Cyclohexan, Halogenkohlenwasserstoffe, wie Methylenchlorid, Chlorbenzol, Dichlorbenzol oder Ether, wie Tetrahydrofuran, Dioxan. Die Reaktionstemperatur liegt im Bereich zwischen 0 und 150°C, vorzugsweise im Bereich zwischen 40 und 120°C. Zweckmäßigerweise setzt man ein wasserabspaltendes Mittel, wie Schwefelsäure, Flußsäure, Polyphosphorsäure, Phosphorpentoxid oder Phosphoroxychlorid zu.

Das Beispiel erläutert die Synthese der Thiadiazolamine:

Zu 25 g ω-4-Chlorphenoxybuttersäure, 10,6 g Thiosemicarbazid und 250 g Dioxan wurden bei 90°C innerhalb einer halben Stunde 6 g Phosphoroxychlorid zugetropft. Dann wurde 1 Stunde lang unter Rühren bei Rückflußtemperatur gehalten, nach dem Abkühlen am Rotationsverdampfer eingengt. Der Rückstand wurde mit Wasser und so viel Natronlauge, daß der pH sich auf etwa 10 einstellt, verrührt. Die

ausgefallenen Kristalle wurden abgesaugt und getrocknet. Die so erhaltene weiße Substanz hat einen Schmelzpunkt von 188 bis 190°C und folgende Struktur:

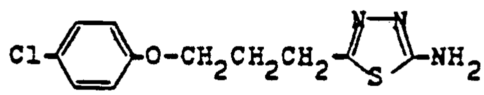

Entsprechend wurden folgende Thiadiazolamine der Formel II hergestellt:

| A | $Z_n$ | $X_m$ | $R^1$ | Fp [°C] |
|---|---|---|---|---|
| $—CH_2—$ | O | 4-Cl | $CH_3$ | 138—140 |
| „ | O | 4-Cl | H | 215—217 |
| „ | — | 4-Phenyl | $CH_3$ | 165—167 |
| „ | — | 4-Phenyl | H | 229—231 |
| „ | — | H | $CH_3$ | 102—104 |
| „ | — | H | H | 178—180 |
| $—(CH_2)_3—$ | O | 4-Cl | $CH_3$ | 105—107 |
| „ | O | 4-Cl | H | 188—190 |
| „ | — | H | $CH_3$ | 56—58 |
| „ | — | H | H | 180—182 |
| $—(CH_2)_4—$ | O | 4-Cl | $CH_3$ | 83—85 |
| „ | O | 4-Cl | H | 157—160 |
| $—(CH_2)_2—$ | O | 4-t.-$C_4H_9$ | $CH_3$ | 114—116 |
| $—(CH_2)_3—$ | O | 4-t.-$C_4H_9$ | H | 194—196 |
| $—(CH_2)_3—$ | O | H | $CH_3$ | 88—90 |
| „ | O | H | H | |
| „ | O | 4-$C_2H_5$ | H | 178—180 |
| „ | O | 4-$CH_3O$ | H | 186—188 |
| „ | O | 4-Br | H | 200—202 |
| „ | O | 3-F | H | 172—174 |
| „ | O | 2-Cl | H | 184—186 |
| „ | O | 3,4-$Cl_2$ | H | 164—166 |
| „ | O | 3,5-$Cl_2$ | H | 171—172 |
| „ | O | 4-CN | H | 154—156 |
| $—(CH_2)_2—$ | O | 4-Cl | $CH_3$ | |

| A | $Z_n$ | $X_m$ | $R^1$ | Fp [°C] |
|---|---|---|---|---|
| $-(CH_2)_2-$ | O | 4-Cl | H | |
| $-(CH_2)_3-$ | O | $3\text{-}CH_3$ | H | 180—182 |
| " | O | $3\text{-}CH_3$ | $CH_3$ | 108—110 |
| " | O | 3-Cl | " | 85—86 |
| " | O | $4\text{-}CH_3$ | " | 115—117 |
| " | O | 2-Cl | " | 68—70 |
| " | O | $4\text{-}NO_2$ | " | 135—137 |
| " | O | 4-CN | " | 119—121 |
| " | O | $3\text{-}NO_2$ | " | |
| " | S | H | " | |
| " | S | 4-Cl | " | |
| " | O | 4-Br | " | 138—140 |
| " | O | $4\text{-}SCH_3$ | " | |
| " | O | $4\text{-}SO_2CH_3$ | " | |
| " | O | $4\text{-}OCH_3$ | " | 110—112 |
| " | O | $4\text{-}t\text{-}C_4H_9$ | " | 107—109 |
| " | O | $4\text{-}C_2H_5$ | " | 100—102 |
| " | O | $4\text{-}CF_3$ | " | |
| " | O | $3\text{-}CF_3$ | " | 78—80 |
| " | O | $3,5\text{-}Cl_2$ | " | 89—90 |
| " | O | $3,4\text{-}Cl_2$ | " | 103—105 |

| A | $Z_n$ | $X_m$ | $R^1$ | Fp [°C] |
|---|---|---|---|---|
| $-(CH_2)_3-$ | O | 4-$C_5H_9$ | $CH_3$ | |
| „ | O | 4-$C_6H_{11}$ | „ | |
| „ | O | 4-(4'-Chlorphenoxy) | „ | |
| „ | O | 4-(2',4'-Dichlorphenoxy) | „ | |
| „ | O | 4-Phenoxy | „ | |
| „ | O | 3-$OC_2H_5$ | „ | |
| „ | O | 2-$CH_3$ | „ | 84—86 |
| „ | O | 4-$OCHF_2$ | „ | |
| „ | O | 3-$OCF_2CHFCl$ | „ | |
| „ | O | 4-S—$C_4H_9$ | „ | |
| $-CH_2-CH_2-CH(CH_3)-$ | O | 4-Cl | „ | |
| $-(CH_2)_3-$ | O | 3-F | „ | 72—74 |
| „ | O | 4-F | „ | |
| „ | O | 2-F | „ | |
| „ | O | 4-J | „ | |
| $-CH(CH_3)CH_2CH_2-$ | O | 4-Cl | „ | 109—111 |
| $-(CH_2)_3-$ | O | 4-$CH_3$ | H | 197—200 |
| „ | O | 3-Cl | H | 149—150 |
| „ | O | 4-Cl | $C_2H_5$ | 98—99 |
| „ | O | 4-Cl | $C_3H_7$ | |
| „ | O | 4-Cl | i-$C_3H_7$ | |
| „ | O | 4-Cl | $C_4H_9$ | |
| „ | O | 4-Cl | t-$C_4H_9$ | |
| $-CH(CH_3)CH_2-$ | — | H | $CH_3$ | 83—85 |
| $-(CH_2)_3-$ | O | 2,4-$Cl_2$ | „ | 118—120 |
| $-CH_2-$ | O | 2-$CH_3$, 4-Cl | „ | 120—122 |
| $-(CH_2)_3-$ | O | 2-$CH_3$, 4-Cl | „ | 134—138 |
| $-(CH_2)_4-$ | O | H | „ | 98—100 |
| „ | O | 2-$CH_3$ | „ | 69—71 |
| „ | O | 2-$CH_3$, 4-Cl | „ | 74—76 |
| „ | O | 2,4-$Cl_2$ | „ | 74—76 |

| A | $Z_n$ | $X_m$ | $R^1$ | Fp [°C] |
|---|---|---|---|---|
| $-(CH_2)_4-$ | O | 3,4-$Cl_2$ | " | 80—82 |
| $-(CH_2)_3-$ | — | 4-$OCH_3$ | " | 79—81 |
| $-(CH_2)_4-$ | O | 2,4,5-$Cl_3$ | $CH_3$ | |
| $-CH_2-$ | O | 4-$NO_2$ | " | |
| " | — | 3-$CH_3$ | " | 95—96 |
| $-C(CH_3)_2-$ | O | 2-$CH_3$ | " | |
| $-(CH_2)_2-$ | O | H | " | 98—101 |
| $-CH(CH_3)CH_2-$ | — | 4-$NO_2$ | " | 132—135 |
| $-C(CH_3)_2-$ | — | 4-$CH_3$ | " | 132—135 |
| $-CH(C_2H_5)-$ | — | H | " | 99—100 |
| $-(CH_2)_6-$ | — | H | " | |
| " | O | H | " | |
| $-CH_2-$ | S | 4-Cl | " | |

| A | $Z_n$ | 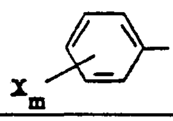 | $R^1$ | Fp [°C] |
|---|---|---|---|---|
| $-(CH_2)_3-$ | O | α-Naphthyl | $CH_3$ | 108—110 |
| " | O | β-Naphthyl | " | 114—116 |
| " | O | α-Naphthyl | H | |
| " | O | 6-Methyl-α-naphthyl | $CH_3$ | |
| " | O | 4,6-Dimethyl-α-naphthyl | $CH_3$ | |
| " | O | β-Naphthyl | H | 176—178 |

Die folgenden Beispiele erläutern die Herstellung der herbiziden Thiadiazolderivate der Formel I:

Beispiel 1

Eine Mischung aus 15 g 2-(3-Phenylpropyl)-5-amino-1,3,4-thiadiazol, 8,4 g Natriumhydrogencarbamat und 200 g Tetrahydrofuran wurde bei 20°C mit 6,5 g Propionylchlorid versetzt und 12 Stunden bei 20°C gerührt. Nach dem Abfiltrieren und Eindampfen wurde der Rückstand mit Petrolether verrührt. Es wurde eine weiße Substanz vom Schmelzpunkt 167—169°C mit folgender Strukturformel erhalten:

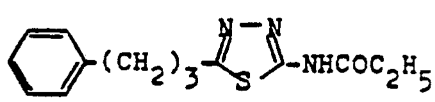

Beispiel 2

Eine Mischung aus 15 g 2-[3-(4'-Chlorphenoxy)-propyl]-5-methylamino-1,3,4-thiadiazol, 3 g Methylisocyanat, 2 Tropfen Dibutylzinndiacetat und 200 g Tetrahydrofuran wurden auf Rückflußtemperatur erwarmt. Nach 4 Stunden wurde eingeengt und der Rückstand wurde mit Diethylether verrieben und abgesaugt. Es wurde eine weiße Substanz mit einem Schmelzpunkt von 83—85°C und folgender Struktur erhalten:

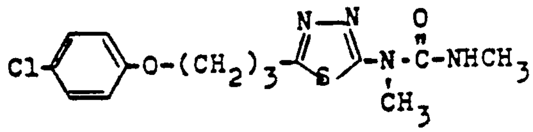

Entsprechend lassen sich folgende Thiadiazolderivate herstellen:

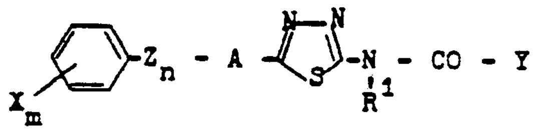

| Nr. | Y | $R^1$ | $-Z_n-A-$ | $X_m$ | Fp [°C] |
|---|---|---|---|---|---|
| 3 | $NHCH_3$ | $CH_3$ | $-O-CH_2-$ | 4-Cl | 146—149 |
| 4 | ,, | ,, | $-CH_2-$ | 4-Phenyl | 152—154 |
| 5 | ,, | ,, | $-O-(CH_2)_4-$ | 4-Cl | 82—84 |
| 6 | ,, | ,, | $-O-(CH_2)_2-$ | 4-Cl | 95—97 |
| 7 | ,, | ,, | $-(CH_2)_3-$ | H | 136—138 |
| 8 | ,, | ,, | $-(CH_2)_2-$ | H | 152—154 |
| 9 | ,, | ,, | $-O-CH(CH_3)CH_2CH_2-$ | 4-Cl | 72—75 |
| 10 | ,, | ,, | $-O-CH_2CH_2CH(CH_3)-$ | 4-Cl | |
| 11 | ,, | ,, | $-S-(CH_2)_3-$ | 4-Cl | |
| 12 | ,, | ,, | $-O-(CH_2)_3-$ | 2-$CH_3$ | 120—123 |
| 13 | ,, | ,, | ,, | 3-$CH_3$ | 73—75 |
| 14 | ,, | ,, | ,, | 4-$CH_3$ | 106—108 |
| 15 | ,, | ,, | ,, | 2-$CH_3$, 4-Cl | 107—109 |
| 16 | ,, | ,, | ,, | 2,4-$Cl_2$ | 125—127 |
| 17 | ,, | ,, | ,, | 2,4,5-$Cl_3$ | |
| 18 | ,, | ,, | ,, | 3,4-$Cl_2$ | 127—129 |
| 19 | ,, | ,, | ,, | 3,5-$Cl_2$ | 150—152 |
| 20 | ,, | ,, | ,, | 2-Cl | 133—135 |
| 21 | ,, | ,, | ,, | 3-Cl | 140—142 |
| 22 | ,, | ,, | ,, | 4-$NO_2$ | 172—174 |
| 23 | ,, | ,, | ,, | 4-CN | 169—171 |
| 24 | ,, | ,, | ,, | 4-Br | 137—139 |

| Nr. | Y | $R^1$ | $-Z_n-A-$ | $X_m$ | Fp [°C] |
|---|---|---|---|---|---|
| 24 | $NHCH_3$ | $CH_3$ | $-O(CH_2)_3-$ | 3-F | |
| 26 | " | " | " | 4-F | |
| 27 | " | " | " | 4-$SCH_3$ | |
| 28 | " | " | " | 4-$SO_2CH_3$ | |
| 29 | " | " | " | 4-$OCH_3$ | 112—114 |
| 30 | " | " | " | 3-$OC_2H_5$ | |
| 31 | " | " | " | H | 114—115 |
| 32 | " | " | " | 4-t-$C_4H_9$ | 132—133 |
| 33 | " | " | " | 4-$C_2H_5$ | 80—82 |
| 34 | " | " | " | 4-$CF_3$ | |
| 35 | " | " | " | 3-$CF_3$ | 100—102 |
| 40 | " | " | " | 4-Cyclopentyl | |
| 41 | " | " | " | 4-Cyclohexyl | |
| 42 | " | " | " | 4-(4′-Chlorphenoxy) | |
| 43 | " | " | " | 4-(2′,4′-Dichlorphenoxy) | |
| 44 | " | " | " | 4-$OCHF_2$ | |
| 45 | " | " | " | 3-$OCF_2CHFCl$ | |
| 46 | " | " | " | 4-$SC_4H_9$ | |
| 47 | " | " | " | 3-$NO_2$ | |
| 48 | " | H | $-OCH_2-$ | 4-Cl | 251—253 |
| 49 | " | " | $-CH_2-$ | 4-Phenyl | 240—245 |
| 50 | " | " | $-O(CH_2)_4-$ | 4-Cl | 186—188 |

| Nr. | Y | $R^1$ | —$Z_n$—A— | $X_m$ | Fp [°C] |
|---|---|---|---|---|---|
| 51 | $NHCH_3$ | H | —O—$(CH_2)_3$— | 4-Cl | 175—177 |
| 52 | „ | „ | —O—$(CH_2)_2$— | 4-Cl | |
| 53 | „ | „ | —$(CH_2)_3$— | H | 174—176 |
| 54 | „ | „ | —$(CH_2)_2$— | H | 162—165 |
| 55 | „ | „ | —O—$(CH_2)_3$— | 3-$CH_3$ | 147—148 |
| 56 | „ | „ | „ | 4-$CH_3$ | 172—174 |
| 57 | „ | „ | „ | 3-Cl | 143—144 |
| 58 | „ | „ | „ | 2-$CH_3$, 4-Cl | |
| 59 | „ | „ | „ | 2,4-$Cl_2$ | |
| 60 | „ | „ | „ | 2,4,5-$Cl_3$ | |
| 62 | „ | „ | —$OCH(CH_3)CH_2CH_2$— | 4-Cl | |
| 63 | „ | „ | —$OCH_2CH_2CH(CH)_3$— | 4-Cl | |
| 64 | $N(OCH_3)CH_3$ | „ | „ | 4-Cl | |
| 65 | „ | „ | —$O(CH_2)_3$— | 4-Cl | 117—119 |
| 66 | „ | „ | „ | 4-$CH_3$ | |
| 67 | „ | „ | „ | 3-Cl | |
| 68 | „ | „ | „ | 4-$CH_3$ | |
| 69 | „ | „ | —O—$(CH_2)_4$— | 4-Cl | 109—111 |
| 70 | „ | „ | —$(CH_2)_3$— | H | Öl |
| 71 | „ | „ | —$(CH_2)_2$— | H | 96—98 |
| 72 | „ | „ | —$OCH_2$— | 4-Cl | 159—161 |
| 73 | $C_2H_5$ | „ | „ | 4-Cl | 223—225 |

| Nr. | Y | $R^1$ | $-Z_n-A-$ | $X_m$ | Fp [°C] |
|---|---|---|---|---|---|
| 74 | $N(OCH_3)CH_3$ | $CH_3$ | $-O-(CH_2)_4-$ | 4-Cl | 86—88 |
| 75 | „ | „ | $-(CH_2)_3-$ | H | Öl |
| 76 | „ | „ | $-(CH_2)_2-$ | H | Öl |
| 77 | „ | „ | $-OCH_2-$ | 4-Cl | 103—105 |
| 78 | „ | „ | $-O(CH_2)_3-$ | 2-Br, 4-Cl | |
| 79 | „ | „ | „ | 2,4-$Cl_2$ | |
| 80 | „ | „ | „ | 3-Cl | |
| 81 | „ | „ | „ | 2-$CH_3$, 4-Cl | |
| 82 | „ | „ | „ | 3-$CH_3$ | |
| 83 | „ | „ | „ | 4-$CH_3$ | |
| 84 | „ | „ | „ | 3-F | |
| 85 | $C_2H_5$ | „ | „ | 4-Cl | 112—114 |
| 86 | „ | „ | $-O(CH_2)_4-$ | 4-Cl | 102—104 |
| 87 | „ | „ | $-CH_2-$ | 4-Phenyl | 115—117 |
| 88 | „ | „ | $-OCH_2-$ | 4-Cl | 118—120 |
| 89 | „ | „ | $-(CH_2)_3-$ | H | 75—77 |
| 90 | „ | „ | $-(CH_2)_2-$ | H | 79—81 |
| 91 | „ | H | „ | H | 172—175 |
| 92 | „ | „ | $-O(CH_2)_3-$ | 4-Cl | |
| 93 | „ | „ | $-O(CH_2)_4-$ | 4-Cl | 198—200 |
| 94 | „ | „ | $-CH_2-$ | 4-Phenyl | 231—233 |
| 95 | Cyclopropyl | „ | $-O(CH_2)_3-$ | 4-Cl | |

| Nr. | Y | $R^1$ | $—Z_n—A—$ | $X_m$ | Fp [°C] |
|---|---|---|---|---|---|
| 96 | $C_3H_7$ | H | $—O—(CH_2)_3—$ | 4-Cl | |
| 97 | $t\text{-}C_4H_9$ | " | " | 4-Cl | |
| 98 | $sec\text{-}C_4H_9$ | " | " | 4-Cl | |
| 99 | $SCH_3$ | " | " | 4-Cl | |
| 100 | $OCH_3$ | " | " | " | |
| 101 | $SC_2H_5$ | " | " | " | |
| 102 | $OC_2H_5$ | " | " | " | |
| 103 | $O\text{-}1\text{-}C_3H_7$ | " | " | " | |
| 104 | $N(CH_3)_2$ | " | " | " | |
| 105 | $NHC_2H_5$ | " | " | " | |
| 106 | $N(CH_3)C_4H_9$ | " | " | " | |
| 107 | $N(CH_3)CH(CH_3)C{\equiv}CH$ | " | " | " | |
| 108 | $N(OC_2H_5)CH_3$ | " | " | " | |
| 109 | $N(OC_3H_7)C_3H_7$ | " | " | " | |
| 110 | 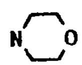 | " | " | " | |
| 111 | 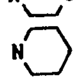 | " | " | " | |
| 112 | 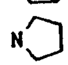 | " | " | " | |
| 113 | 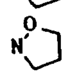 | " | " | " | |
| 114 | 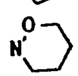 | " | " | " | |
| 115 | 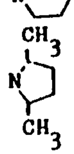 | " | " | " | |

| Nr. | Y | $R^1$ | $—Z_n—A—$ | $X_m$ | Fp [°C] |
|---|---|---|---|---|---|
| 116 | $NHC_2H_5$ | $CH_3$ | $—O—(CH_2)_3—$ | 4-Cl | 113—116 |
| 117 | $NHC_3H_7$ | " | " | " | |
| 118 | $NHcycC_3H_5$ | " | " | " | |
| 119 | $NHCH_2CH{=}CH_2$ | " | " | " | |
| 120 | $NHCH_2C{\equiv}CH$ | " | " | " | |
| 121 | $NHCH_2CH_2OCH_3$ | " | " | " | |
| 122 | Cyclopentylamino | " | " | " | |
| 123 | $NHCH_3$ | $C_2H_5$ | " | " | 120—122 |
| 124 | $NHCH_3$ | t-$C_4H_9$ | " | " | |
| 125 | $NHCH_3$ | $CH_3$ | " | 4-J | |
| 126 | " | " | $—CH(CH_3)CH_2—$ | H | |
| 127 | " | " | $—OCH_2—$ | 2-$CH_3$, 4-Cl | 186—188 |
| 128 | " | " | " | 4-$NO_2$ | |
| 129 | " | " | $—SCH_2—$ | 4-Cl | |
| 130 | " | " | $—OCH_2—$ | 2,4-$Cl_2$ | |
| 131 | " | " | $—OCH(CH_3)—$ | 2,4-$Cl_2$ | |
| 132 | " | " | $—OCH(CH_3)—$ | 4-(2',4'-Dichlorphenoxy) | |
| 133 | " | " | $—O(CH_2)_4—$ | H | 87—89 |
| 134 | " | " | " | 2-$CH_3$ | 112—114 |
| 135 | " | " | " | 2-$CH_3$, 4-Cl | 116—118 |
| 136 | " | " | " | 2,4-$Cl_2$ | 127—129 |
| 137 | " | " | " | 3,4-$Cl_2$ | 98—100 |
| 138 | " | " | $—(CH_2)_3—$ | 4-$OCH_3$ | 87—88 |
| 139 | " | " | $—O(CH_2)_4—$ | 2,4,5-$Cl_3$ | |

| Nr. | Y | $R^1$ | $—Z_n—A—$ | $X_m$ | Fp [°C] |
|---|---|---|---|---|---|
| 140 | $NHCH_3$ | $CH_3$ | $—CH_2—$ | $3-CH_3$ | 166—168 |
| 141 | „ | „ | $—OC(CH_3)_2—$ | $2-CH_3$ | |
| 142 | „ | „ | $—O(CH_2)_2—$ | H | 168—170 |
| 143 | „ | „ | $—CH(CH_3)CH_2—$ | $4-NO_2$ | 153—155 |
| 144 | „ | „ | $—(CH_2)_6—$ | H | |
| 145 | „ | „ | $—O(CH_2)_6—$ | H | |
| 146 | „ | „ | $—SCH_2—$ | H | |
| 147 | „ | „ | $—OCH(CH_3)—$ | $3-t-C_4H_9$ | |
| 148 | „ | „ | $—OCH(CH_3)—$ | $2,4,5-Cl_3$ | |
| 149 | „ | „ | $—OCH(CH_3)—$ | $2-CH_3, 4,5-Cl_2$ | |
| 150 | „ | „ | $—O(CH_2)_7—$ | 4-Cl | |
| 151 | $OCH_3$ | H | $—(CH_2)_3—$ | H | 178—180 |
| 152 | $SCH_3$ | $CH_3$ | $—OCH_2—$ | 4-Cl | 108—110 |
| 153 | $OCH_3$ | „ | „ | „ | 114—116 |
| 154 | $NHCH_3$ | „ | $—C(CH_3)_2CH_2—$ | $4-CH_3$ | 127—130 |
| 155 | „ | „ | „ | H | 133—135 |
| 156 | „ | „ | $—CH(C_2H_5)—$ | H | 124—125 |
| 157 | „ | H | $—O(CH_2)_3—$ | $4-OCH_3$ | 137—138 |
| 158 | „ | „ | „ | 4-Br | 162—163 |
| 159 | „ | „ | „ | 2-Cl | 175—177 |
| 160 | „ | „ | „ | $4-C_2H_5$ | 165—167 |
| 161 | „ | „ | „ | 3-F | 140—141 |

| Nr. | Y | $R^1$ | $-Z_n-A-$ | $X_m$ | Fp [°C] |
|---|---|---|---|---|---|
| 162 | $NHCH_3$ | $CH_3$ | $-O(CH_2)_2-$ | 4-t-$C_4H_9$ | 119—121 |
| 167 | $C_2H_5$ | H | $-O(CH_2)_3-$ | 3-$CH_3$ | 148—150 |
| 168 | „ | „ | „ | 4-t-$C_4H_9$ | 156—158 |
| 169 | „ | „ | „ | 4-CN | 225—230 |
| 170 | „ | „ | „ | 2-Cl | 162—163 |
| 171 | „ | „ | „ | 3,4-$Cl_2$ | 170—172 |
| 172 | „ | „ | „ | 3,5-$Cl_2$ | 193—195 |
| 173 | $NHCH_3$ | $CH_3$ | $-(CH_2)_4-$ | H | 123—125 |
| 174 | „ | „ | „ | 4-$CH_3$ | 98—100 |
| 175 | „ | „ | „ | 4-$OCH_3$ | 120—122 |
| 176 | „ | „ | „ | 4-Cl | 89—91 |
| 177 | „ | „ | „ | 3,4-$Cl_2$ | |
| 178 | „ | „ | $-(CH_2)_5-$ | 4-$CH_3$ | Öl |
| 179 | „ | „ | „ | 4-Cl | |
| 180 | „ | „ | $-O(CH_2)_4-$ | 3-$CH_3$ | 102—104 |
| 182 | „ | „ | „ | 2-Cl | 81—83 |
| 183 | „ | „ | „ | 3,5-$Cl_2$ | 134—136 |
| 184 | „ | „ | „ | 2,5-$Cl_2$ | 115—117 |
| 185 | „ | „ | „ | 3-Br | 105—107 |
| 186 | „ | „ | „ | 3,5-$(CH_3)_2$ | 139—141 |
| 187 | „ | „ | „ | 3-Cl | 87—89 |
| 188 | „ | „ | „ | 4-Br | 105—106 |

| Nr. | Y | $R^1$ | $-Z_n-A-$ | $X_m$ | Fp [°C] |
|---|---|---|---|---|---|
| 189 | $NHCH_3$ | $CH_3$ | $-O(CH_2)_4-$ | $3,4\text{-}(CH_3)_2$ | 111—113 |
| 190 | „ | H | „ | $2\text{-}CH_3$ | 136—137 |
| 191 | „ | $CH_3$ | $-OCH_2-$ | $2,4\text{-}Cl_2$ | 173—176 |
| 192 | „ | „ | $-O(CH_2)_5-$ | $3,4\text{-}Cl_2$ | 108—110 |
| 193 | „ | H | $-O(CH_2)_4-$ | $3\text{-}CH_3$ | 67—68 |
| 194 | $CH_3$ | $CH_3$ | „ | $3,4\text{-}Cl_2$ | 106—108 |
| 195 | $C_2H_5$ | „ | „ | $3,4\text{-}Cl_2$ | 102—104 |
| 196 | $CH_3$ | „ | $-CH_2-$ | $3\text{-}CH_3$ | 78—79 |
| 197 | $C_2H_5$ | „ | „ | $3\text{-}CH_3$ | 77—78 |
| 198 | $NHCH_3$ | „ | „ | 4-Cl | 197—199 |
| 199 | „ | „ | „ | $4\text{-}OCH_3$ | 175—177 |
| 200 | „ | „ | „ | $4\text{-}CH_3$ | 202—203 |
| 201 | „ | „ | „ | H | 188—190 |
| 202 | „ | „ | „ | $2\text{-}OCH_3$ | 208—209 |
| 203 | „ | „ | „ | 2-Cl | 204—206 |
| 204 | „ | „ | „ | $2,4\text{-}Cl_2$ | 160—162 |
| 205 | „ | „ | „ | $3\text{-}CF_3$ | 119—121 |
| 206 | „ | „ | „ | $3\text{-}OCH_3$ | 138—140 |
| 207 | $N(OCH_3)CH_3$ | „ | „ | $3\text{-}CH_3$ | 70—72 |
| 208 | $NH(n\text{-}C_4H_9)$ | „ | „ | $3\text{-}CH_3$ | 91—93 |
| 209 | $NH(n\text{-}C_4H_9)$ | „ | $-O(CH_2)_3-$ | 4-Cl | 82—85 |
| 210 | $NH(CH_2OCH_3)$ | „ | „ | 4-Cl | 86—88 |

| Nr. | Y | $R^1$ | $-Z_n-A-$ | $X_m$ | Fp [°C] |
|---|---|---|---|---|---|
| 211 | $NH(t\text{-}C_4H_9)$ | $CH_3$ | $-O(CH_2)_3-$ | 4-Cl | 98—100 |
| 212 | $N(OCH_3)CH_3$ | " | " | 4-Cl | 106—108 |
| 213 | $NH(C(CH_3)_2C{\equiv}CH)$ | " | " | 4-Cl | 88—91 |
| 214 | $NHCH_3$ | $CH_3$ | $-CH_2CH(CH_3)-$ | $4\text{-}t\text{-}C_4H_9$ | 113—114 |
| 215 | $NHCH_3$ | $C_2H_5$ | $-O(CH_2)_4-$ | $2\text{-}CH_3$ | 89—90 |
| 216 | $NHCH_3$ | $C_2H_5$ | " | H | 92—94 |
| 217 | $NHCH_3$ | $C_2H_5$ | " | $3,4\text{-}Cl_2$ | 75—78 |
| 218 | $NHCH_3$ | H | " | $3,4\text{-}Cl_2$ | 177—178 |
| 219 | $NHCH_3$ | $CH_3$ | $-SCH_2-$ | $2,5\text{-}(CH_3)_2$, 4-Cl | 173—174 |
| 220 | $NHCH_3$ | $CH_3$ | $-OCH(CH_3)$ | $2\text{-}CH_3$, 4-Cl | 47—48 |

| Nr. | Y | $R^1$ | $-Z_n-A-$ | 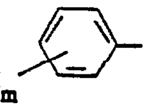 | Fp [°C] |
|---|---|---|---|---|---|
| 36 | $NHCH_3$ | $CH_3$ | $-O-(CH_2)_3-$ | α-Naphthyl | 100—102 |
| 37 | $NHCH_3$ | „ | „ | β-Naphthyl | 112—114 |
| 38 | $NHCH_3$ | „ | „ | 2-Methyl-α-naphthyl | |
| 39 | $NHCH_3$ | „ | „ | 2,4-Dimethyl-α-naphthyl | |
| 61 | $NHCH_3$ | H | „ | 4-Chlor-α-naphthyl | |
| 163 | $NHCH_3$ | H | „ | α-Naphthyl | |
| 164 | $NHCH_3$ | H | „ | β-Naphthyl | |
| 165 | $C_2H_5$ | H | „ | α-Nahthyl | >240 |
| 166 | $C_2H_5$ | H | „ | β-Naphthyl | 162—164 |
| 181 | $NHCH_3$ | $CH_3$ | $-O-(CH_2)_4-$ | α-Naphthyl | 110—112 |

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen, Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 8 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 7 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kiesel-säuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 6 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 51 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 1 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe für die Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Bekämpfungsziel und Wachstumsstadium 0,05 bis 10 kg/ha und mehr, vorzugsweise 0,1 bis 5 kg/ha.

Die herbizide Wirkung von Verbindungen der Formel I wurde durch Gewächshausversuche gezeigt.

Also Kulturgefäße dienten Plastikblumentöpfe mit 300 cm$^3$ Inhalt und lehmigem Sand mit etwa 1,5% Humus als Substrat. Die Samen der Testpflanzen wurden nach Arten getrennt flach eingesät. Bei Vorauflaufanwendung wurden die Wirkstoffe unmittlebar danach auf die Erdoberfläche aufgebracht. Sie wurden hierzu in Wasser als Verteilungsmittel suspendiert oder emulgiert und mittels fein verteilender Düsen gespritzt. Die Augwandmengen betrugen 3,0 kg Wirkstoff/ha. Nach dem Aufbringen der Mittel wurden die Gefäße leicht beregnet, um Keimung und Wachstum in Gang zu bringen. Danach wurden die Gefäße mit durchsichtigen Plastickhauben abgedeckt, bis die Pflanzen angewachsen waren. Diese Abdeckung bewirkte ein gleichmäßiges Keimen der Testpflanzen, sofern diese nicht durch die Wirkstoffe beeinträchtigt wurden.

Zum Zwecke der Nachauflaufbehandlung zog man die Testpflanzen je nach Wuchsform bis zu einer Wuchshöhe von 3 bis 15 cm an und behandelte sie danach. Die für die Nachauflaufanwendung benutzten Sojapflanzen zog man in einem mit Torfmull (peat) angereichertem Substrat an. Zur Nachauflaufbehandlung wurden entweder direkt gesäte und in den gleichen Gefäßen aufgewachsene Pflanzen ausgewählt, oder sie wurden erst als Keimplanzen getrennt angezogen une einige Tage vor der Behandlung in die Versuchsgefäße verpflanzt. Die Aufwandmengen für die Nachauflaufbehandlung variierten je nach Wirkstoff. Sie betrugen 0,125, 0,25, 0,5, 1,0 oder 3,0 kg Wirkstoff/ha. Die Abdeckung unterblieb bei der Nachauflaufbehandlung.

Die Versuchsgefäße wurden im Gewächshaus aufgestellt, wobei für wärmeliebende Arten wärmere Bereiche (20 bis 35°C) und für solche gemäßigter Klimate 10 bis 25°C bevorzugt wurden. Die Versuchsperiode erstreckte sich über 2 bis 4 Wochen. Während dieser Zeit wurden die Pflazen gepflegt, und ihre Reaktion auf die einzelnen Behandlungen wurde ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die in den Versuchen verwendeten Pflanzen setzen sich aus folgenden Arten zusammen:

Amaranthus spp. (Fuchsschwanzarten), Amaranthus retroflexus (zurückgekrümmter Fuchsschwanz), Avena sativa (Hafer), Beta vulgaris (Zuckerrübe), Cassia tora, Centaurea cyanus (Kornblume), Chenopodium album (Weißer Gänsefuß), Euphorbia geniculata (Südamerik. Wolfsmilch), Galium aparine (Klettenlabkraut), Glycine max. (Sojabohnen), Gossypium hirsutum (Baumwolle), Ipomoea spp. (Prunkwindearten), Lamium spp. (Taubnesselarten), Lolium multiflorum (Ital. Raygras), Setaria spp. (Borsenhisearten), Sinapis alba (Weißer Senf), Solanum nigrum (Schwarzer Nachtschatten), Triticum aestivum (Weizen), Veronica persica (Ehrenpreis), Viola tricolor (Gewöhnliches Stiefmütterchen), Zea mays (Mais), Oryza sativa (Reis).

Bei der Prüfung auf herbizide Wirkung bei Vorauflaufanwendung im Gewächshaus zeigten die Verbindungen Nr. 7, 9, 25, 31 und 156 mit 3,0 kg Wirkstoff/ha eine erhebliche Aktivität.

Bei der Prüfung auf herbizide Wirkung bei Nachauflaufanwendung von 3,0 kg Wirkstoff/ha zeigten die Verbindung Nr. 2, 4, 5, 6, 7, 51, 53, 54, 69, 89, 94 und 152 eine gute Aktivität an den Beispielspflanzen. Die Verbindung Nr. 2 hatte bei Nachauflaufanwendung von 0,25 kg Wirkstoff/ha eine sehr gute herbizide Wirkung gegen zahlreiche breitblättrige unerwünschte Pflanzen.

Bei Nachauflaufanwendung bekämpften die Verbindungen Nr. 9, 69, 152 mit 0,5 kg Wirkstoff/ha unerwünschte Pflanzen in Kulturen selektiv. Breitblättrige Unkräuter wurden in Mais mit 0,125 kg Wirkstoff Nr. 35/ha im Nachauflaufverfahren bekämpft. Außerdem waren die Verbindungen Nr. 18 mit 0,25 kg Wirkstoff/ha sowie die Verbindungen Nr. 32, 37 mit 1,0 kg Wirkstoff/ha selektiv wirksam gegen Unkräuter.

In Anbetracht der Vielseitigkeit der Applikationsmethoden können die erfindungsgemäßen Verbindungen noch in einer weiteren Zahl von Kulturlpflanzen zur Beseitigung unerwünschter Pflanzen eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Brassica napus var. napobrassica | Kohlrübe |
| Brassica napus var. rapa | Weiße Rübe |
| Camellia sinensis | Teestrauch |
| Carthamus tinctorius | Saflor-Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |

| Botanischer Name | Deutscher Name |
|---|---|
| Cynodon dactylon | Bermudagras |
| Daucus carota | Möhre |
| Elaeis guineenis | Ölpalme |
| Fragaria vesca | Erdbeere |
| Glycine max | Sojabohne |
| Gossypium hirsutum (Gossypium arboreum Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotinana tabacum (N. rustica) | Tabak |
| Olea europaea | Ölbaum |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |

| Botanischer Name | Deutscher Name |
|---|---|
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Theobroma cacoa | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen erfindungsgemäßen Verbindungen mit zahlreichen Vertretern anderer herbizider oder wachstumsregulierender Wirkstoffgruppen gemischst und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Eine Reihe von Wirkstoffen, welche zusammen mit den neuen Verbindungen für verschiedenste Anwendungsbereiche sinnvolle Mischungen ergeben, werden beispeilhaft aufgeführt:

3-(1-Methylethyl)-1H-2,1,3-benzothiadiazin-4(3*H*)-on-2,2-dioxid und Salze

3-(1-Methylethyl)-8-chlor-1H-2,1,3-benzothiadiazin-4(3*H*)-on-2,2-dioxid und Salze

3-(1-Methylethyl)-8-fluor-1H-2,1,3-benzothiadiazin-4(3*H*)-on-2,2-dioxid und Salze

3-(1-Methylethyl)-8-methyl-1H-2,1,3-benzothiadiazin-4(3*H*)-on-2,2-dioxid und Salze

1-Methoxymethyl-3-(1-methylethyl)-2,1,3-benzothiadiazin-4(3*H*)-on-2,2-dioxid
1-Methoxymethyl-8-chlor-3-(1-methylethyl)-2,1,3-benzothiadiazin-4(3*H*)-on-2,2-dioxid
1-Methoxymethyl-8-fluor-3-(1-methylethyl)-2,1,3-benzothiadiazin-4(3*H*)-on-2,2-dioxid
1-Cyan-8-chlor-3-(1-methylethyl)-2,1,3-benzothiadiazin-4(3*H*)-on-2,2-dioxid
1-Cyan-8-fluor-3-(1-methylethyl)-2,1,3-benzothiadiazin-4(3*H*)-on-2,2-dioxid
1-Cyan-8-methyl-3-(1-methylethyl)-2,1,3-benzothiadiazin-4(3*H*)-on-2,2-dioxid
1-Azidomethyl-3-(1-methylethyl)-2,1,3-benzothiadiazin-4(3*H*)-on-2,2-dioxid
3-(1-Methylethyl)-1H-(pyridino-[3,2-e]2,1,3-thiadiazin-(4)-on-2,2-dioxid
N-(1-Ethylpropyl)-2,6-dinitro-3,4-dimethylanilin
N-(1-Methylethyl)-N-ethyl-2,6-dinitro-4-trifluoromethyl-anilin
N-n-Propyl-N-β-chlorethyl-2,6-dinitro-4-trifluoromethyl-anilin
N-n-Propyl-N-cyclopropylmethyl-2,6-dinitro-4-trifluormethyl-anilin
N,N-Di-n-propyl-2,6-dinitro-3-amino-4-trifluoromethylanilin
N,N-Di-n-propyl-2,6-dinitro-4-methyl-anilin
N,N-Di-n-propyl-2,6-dinitro-4-methylsulfonyl-anilin
N,N-Di-n-propyl-2,6-dinitro-4-aminosulfonyl-anilin
N,N-Di-beta-chlorethyl-2,6-dinitro-4-methyl-anilin
N-Ethyl-2-(2-methylallyl)-2,6-dinitro-4-trifluormethyl-anilin
N-Methylcarbaminsäure-3,4-dichlorbenzylester
N-Methylcarbaminsäure-2,6-di-tert.butyl-4-methylphenylester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
Ethyl-N-[3-(N'-phenylcarbamoyloxy)-phenyl]-carbamat
Methyl-N-[3-(N'-methyl-N'-phenylcarbamoyloxy)-phenyl]-carbamat
Isopropyl-N-[3-(N'-ethyl-N'-phenylcarbamoyloxy)-phenyl]-carbamat
Methyl-N-[3-(N'-3-methylphenylcarbamoyloxy)-phenyl]-carbamat
Methyl-N-[3-(N'-4-fluorphenylcarbamoyloxy)-phenyl]-carbamat
Methyl-N-[3-(N'-3-chlor-4-fluorphenylcarbamoyloxy)-phenyl]-carbamat
Ethyl-N-[3-(N'-3-chlor-4-fluorphenylcarbamoyloxy)-phenyl]-carbamat
Ethyl-N-[3-(N'-3,4-difluorophenylcarbamoyloxy)-phenyl]-carbamat
Methyl-N-[3-(N'-3,4-difluorphenylcarbamoyloxy)-phenyl]-carbamat
N-3-(2-Methylphenoxycarbonylamino)-phenylcarbaminsäure-ethylester
N-3-(4-Fluorphenoxycarbonylamino)-phenylthiolcarbaminsäure-methylester
N-3-(2,4,5-Trimethylphenoxycarbonylamino)-phenylthiolcarbaminsäure-methylester
N-3-(Phenoxycarbonylamino)-phenylthiolcarbaminsäure-methylester
N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo[2.2.1]heptyl-thiolcarbaminsäureethylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1H-azepin-1-carbothiolat
S-Benzyl-3-methylhexahydro-1H-azepin-1-carbothiolat
S-Benzyl-2,3-dimethylhexahydro-1-H-azepin-1-carbothiolat
S-Ethyl-3-methylhexahydro-1-H-azepin-1-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
Alpha,alpha-Dichlorpropionsäure-Natriumsalz
Alpha,alpha-Dichlorbuttersäure-Natriumsalz

Alpha,alpha,beta,beta-Tetrafluorpropionsäure-Natriumsalz
Alpha-Methyl-alpha,beta-dichlorpropionsäure-Natriumsalz
Alpha-Chlor-beta-(4-chlorphenyl)-propionsäure-methylester
Alpha,beta-Dichlor-beta-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-Methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz
2-(N-Benzoyl-3-chlor-4-fluorphenylamino)-propionsäuremethylester
2-(N-Benzoyl-3-chlor-4-fluorphenylamino)-propionsäureisopropylester
2-Chlor-4-ethylamino-6-isopropylamino-1,3,5-triazin
2-Chlor-4-ethylamino-6-(amino-2'-propionitril)-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin
2-Azido-4-methylamino-6-isopropylamino-1,3,5-triazin
2-Methylthio-4-ethylamino-6-isopropylamino-1,3,5-triazin.
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin
2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cylcohexyl-6-dimethylamino-1,3,5-triazin-2,4-dion.
3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil
2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl]ethan (Salze)
1-(4-Chlorphenoxy-3,3-dimethyl-1-(H-1,2,4-triazolyl)-2-butanon
N,N-Diallylchloracetamid
N-Isopropyl-2-chloracetanilid
N-(1-Methyl-propin-2-yl)-2-chloracetanilid
2-Methyl-6-ethyl-N-propargyl-2-chloracetanilid
2-Methyl-6-ethyl-N-ethoxymethyl-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazolyl-methyl)-2-chloracetanilid
2-Methyl-6-ethyl-N-(pyrazolyl-methyl)-2-chloracetanilid
2,6-Dimethyl-N-(pyrazolyl-methyl)-2-chloracetanilid
2,6-Dimethyl-N-(4-methylpyrazolyl-methyl)-2-chloracetanilid
2,6-Dimethyl-N-(1,2,4-triazolyl-methyl)-2-chloracetanilid

2,6-Dimethyl-N-(3,5-dimethylpyrazolyl-methyl)-2-chloracetanilid
2,6-Dimethyl-N-(1,3-dioxalan-2-yl-methyl)-2-chloracetanilid
2,6-Dimethyl-N-(2-methoxyethyl)-2-chloracetanilid
2,6-Dimethyl-N-isobutoxymethyl-2-chloracetanilid
2,6-Diethyl-N-methoxymethyl-2-chloracetanilid
2,6-Diethyl-N-(n-butoxymethyl)-2-chloracetanilid
2,6-Diethyl-N-ethoxycarbonylmethyl-2-chloracetanilid
2,3,6-Trimethyl-N-(pyrazolyl-methyl)-2-chloracetanilid
2,3-Dimethyl-N-isopropyl-2-chloracetanilid
2,6-Dimethyl-N-(2-n-propoxy-ethyl)-2-chloracetanilid
2-(alpha-Naphthoxy)-N,N-diethylpropionamid
2,2-Diphenyl-N,N-dimethylacetamid
Alpha-(3,4,5-Tribrompyrazolyl)-N,N-dimethylpropionamid
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid
2-Propionyl-amino-4-methyl-5-chlor-thiazol
O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4-dinitrophenylbenzaldoxim (Salze)
3,5-Dibrom-4-hydroxy-O-(2-cyan-4-nitrophenyl-benzaldoxim (Salze)
Pentachlorphenyl-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluoromethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion
2-(3-Isopropylcarbamoyl-oxyphenyl)-4-methyl-1,2,4-oxadiazolidin-3,5-dion
2-Phenyl-3,1-benzoxazinon-(4)
2-Ethoxy-2,3-dihydro-3,3-dimethyl-5-benzofuranyl-methan-sulfonat
2-Ethoxy-2,3-dihydro-3,3-dimethyl-5-benzofuranyl-dimethylaminosulfonat
2-Ethoxy-2,3-dihydro-3,3-dimethyl-5-benzofuranyl-(N-methyl-N-acetyl)-aminosulfonat
3,4-Dichlor-1,2-benzisothiazol
2-Methyl-4,6-dinitrophenol (Salze, Ester)
2-sec.Butyl-4,6-dinitrophenol (Salze, Ester)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat
2-sec.Amyl-4,6-dinitrophenol (Salze, Ester)
1-(alpha,alpha-Dimethylbenzyl)-3-(4-methylphenyl)-harnstoff
1-Phenyl-3-(2-methylcylcohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimiethyl-harnstoff
1-(4-Chlorphenyl)-3-methyl-3-(butin-1-yl-3)-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff

1-(4-i-Propylphenyl)-3,3-dimethyl-harnstoff
1-(3-Trifluormethylphenyl)-3,3-dimehyl-harnstoff
1-(alpha,alpha,beta,beta-Tetrafluorethoxyphenyl)-3,3-dimethyl-harnstoff
1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-isobutylamid
1,2-Dimethyl-3,5-diphenylpyrazolium-methylsulfat
1,2-4-Trimethyl-3,5-diphenylpyrazolium-methylsufat
1,2-Dimethyl-4-brom-3,5-diphenylpyrazolium-methylsulfat
1,3-Dimethyl-4-(3,4-dichlorbenzoyl)-5-[(4-methylphenylsulfonyl)-oxzy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1-(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)
Alpha-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylate
9-(Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)
2-Chlorethanphosphonsäure-2-chlorethylester

Ammonium-ethyl-carbamoyl-phosphonat
Trithiobutylphosphit
O,O-Diisopropyl-5-(2-benzolsulfonylamino-ethyl)-phosphordithionat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
(2-Chlorethyl)-trimethyl-ammoniumchlorid
(2-Methyl-4-phenylsulfonyl)-trifluormethansulfonanilid
Natriumchlorat
Ammoniumrhodanid
Calciumcyanamid
2-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert.-Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Jodpyridyl-2-oxy)-phenoxy]-propionsäure-n-butylester
2-Chlor-4-trifluormethylphenyl-3'-(2-fluorethoxy)-4'-nitrophenylether
2-Chlor-4-trifluormethylphenyl-3'-ethoxycarbonylmethylthio-4'-nitrophenylether
2,4,6-Trichlorphenyl-3'-ethoxycarbonyl)methylthio-4'-nitrophenylether
2-[1-(N-ethoxyamino)-butyliden]-5-(2-ethylthiopropyl)-3-hydroxy-cyclohexen-(2)-on-(1) (Salze)
2-[1-(N-ethoxyamino)-butyliden]-5-(2-phenylthiopropyl)-3-hydroxy-cyclohexen-(2)-on-(1) (Salze)
4-[4-(4'-Trifluormethyl)-phenoxy]-penten-2-carbonsäureethylester
2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-alpha,alpha,beta-trifluor-beta-bromethoxyphenyl)-3-(2H)-pyridazinon
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1-(3-Chlor-4-ethoxyphenyl)-3,3-dimethylharnstoff
2-Methyl-4-chlorphenoxy-thioessigsäureethylester
2-Chlor-3,5-dijod-4-acetoxy-pyridin
1-(4-[2-(4-Methylphenyl)-ethoxy]-phenyl)-3-methyl-3-methoxyharnstoff
2,6-Dimethyl-N-(pyrazolyl-methylenoxymethyl)-2-chloracetanilid
2-Methyl-6-ethyl-N-(pyrazolyl-methylenoxymethyl)-2-chloracetanilid
1-alpha-2-Brom-4-chlorphenoxypropionsäure)-3-(O-methylcarbamoyl)-anilid
2-Methyl-6-ethyl-N-(pyrazolyl-ethylenoxymethyl)-2-chloracetanilid
Methyl-N-dichlorfluormethylsulfenyl-[3-[N'-dichlorfluormethyl-sulfenyl-N'-phenylcarbamoyl-oxy)-phenyl]-carbamat
Methyl-N-dichlorfluormethylsulfenyl-[3-(N'-dichlorfluormethyl-sulfenyl-N'-3-methylphenylcarbamoyl-oxy)-phenyl]-carbamat
N-(Pyrazolyl-methyl)-pyrazolyl-essigsäure-2,6-dimethylanilid
N-(Pyrazolyl-methyl)-1,2,4-triazolyl-essigsäure-2,6-dimethylanilid
2-(3-Trifluormethylphenyl)-4H-3,1-benzoxazin-4-on
2-(2-Thineyl)-4H-3,1-benzoxazin-4-on
2-(3-Pentafluorethoxyphenyl)-4H-3,1-benzoxazin-4-on
2-(3-Trifluormethylthio-phenyl)-4H-3,1-benzoxazin-4-on
2-(3-Difluorchlormethoxyphenyl)-4H-3,1-benzoxazin-4-on
5-Nitro-2-(3-trifluormethyl-phenyl)-4H-3,1-benzoxazin-4-on
5-Chlor-2-(3-trifluormethoxyphenyl)-4H-3,1-benzoxazin-4-on
5-Chlor-2-(3-alpha,alpha,beta,beta-tetrafluorethoxyphenyl)-4H-3,1-benzoxazin-4-on
5-Fluor-2-(3-alpha,alpha,beta,beta-tetrafluorethoxyphenyl)-4H-3,1-benzoxazin-4-on
5-Chlor-2-(4-difluorchlormethoxyphenyl)-4H-3,1-benzoxazin-4-on
5-Fluor-2-(4-difluorchlormethoxyphenyl)-4H-3,1-benzoxazin-4-on
5-Fluor-2-phenyl-4H-3,1-benzoxazin-4-on
5-Fluor-2-(3-difluormethoxyphenyl)-4H-3,1-benzoxazin-4-on
5-Chlor-2-phenyl-4H-3,1-benzoxazin-4-on
3-(3,5-Dichlorphenyl)-4-methoxycarbonyl-5-methylpyrazol
3-(3-Chlorphenyl)-4-methoxycarbonyl-5-methylpyrazol
3-(3-Fluorphenyl)-4-methoxycarbonyl-5-methylpyrazol
1-Acetyl-3-(3-fluorphenyl)-4-methoxycarbonyl-5-methylpyrazol

1-Acetyl-3-(3-chlorphenyl)-4-methoxycarbonyl-5-methylpyrazol
1-Acetyl-3-(3-bromphenyl)-4-methoxycarbonyl-5-methylpyrazol
1-Acetyl-3-(3,5-dichlorphenyl)-4-methoxycarbonyl-5-methylpyrazol
1-Acetyl-3-thienyl-4-methoxy-carbonyl-5-methylpyrazol
N-3-Chlor-4-isopropylphenyl-thiolcarbaminsäuremethylester
N-3-Methyl-4-fluorphenyl-thiolcarbaminsäuremethylester
N-3-Chlor-4-isopentyl-phenyl- thiolcarbaminsäuremethylester
N-3-Chlor-4-difluormethoxyphenyl-thiolcarbaminsäuremethylester
N-3-Chlor-4-[(1-chlorisopropyl)-phenyl]-thiocarbaminsäuremethylester
1-(2-Fluorphenyl)-3-methyl-5-iminolimidazolidin-2-on
1-(3-Isopropyl-phenyl)-3-methyl-5-iminoimidazolidin-2-on
1-(4-Isopropyl-phenyl)-3-methyl-5-iminoimidazolidin-2-on
1-[3-(1,1,2,2-Tetrafluor-ethoxy)-phenyl]-3-methyl-5-iminoimidazolidin-2-on
1-(3,4-Dichlorphenyl)-3-methyl-5-iminoimidazolidin-2-on
1-(3,4-Difluorphenyl)-3-methyl-5-iminoimidazolidin-2-on
6-Methyl-3-methoxy-5,6-dihydro-1,2,4,6-thiatriazin-5-on-1,1-dioxid
6-Methyl-3-methoxy-5,6-dihydro-1,2,4,6-thiatriazin-5-on-1,1-dioxid Natriumsalz
6-n-Propyl-3-methoxy-5,6-dihydro-1,2,4,6-thiatriazin-5-on-1,1-dioxid
6-Methyl-3-ethoxy-5,6-dihydro-1,2,4,6-thiatriazin-5-on-1,1-dioxid
6-n-Propyl-3-ethoxy-5,6-dihydro-1,2,4,6-thiatriazin-5-on-1,1-dioxid Natriumsalz
6-Methyl-3-iso-propoxy-5,6-dihydro-1,2,4,6-thiatriazin-5-on-1,1-dioxid
6-n-Propyl-3-iso-propoxy-5,6-dihydro-1,2,4,6-thiatriazin-5-on-1,1-dioxid
6-Isopropyl-3-sek.-butoxy-4,6-dihydro-1,2,4,6-thiatriazin-5-on-1,1-dioxid Natriumsalz
N-3'-(2''-Chlor-4''-trifluormethyl-phenoxy)-6'-nitrobenzoylanthranilsäure
N-3'-(2''-Chlor-4''-trifluormethyl-phenoxy)-6'-nitrobenzoylanthranilsäure-methylester
N-3'-(2''-Chlor-4''-trifluormethyl-phenoxy)-6'-nitrobenzoylanthranilsäure-Natriumsalz
N-3'-(2''-Chlor-4''-trifluormethyl-phenoxy)-6'-nitrobenzoyl-3-chloranthranilsäure
N-3'-(2''-Chlor-4''-trifluormethyl-phenoxy)-benzoyl-3-chloranthranilsäure
N-3'-(2''-Chlor-4''-trifluormethyl-phenoxy)-benzoyl-3-methylanthranilsäure
N-3'-(2''-Chlor-4''-trifluormethyl-phenoxy)-benzoylanthranilsäure
N-3'-(2''-Dichlorphenoxy)-6'-nitrobenzoylanthranilsäure
N-[3'-(2''-Chlor-4''-trifluormethyl-phenoxy)-6'-nitrophenyl]-4H-1,3-benzoxazin-4-on
N-[3'-(2''-Chlor-4''-trifluormethyl-phenoxy)-6'-nitrophenyl]-4H-1,3-8-methoxy-benzoxazin-4-on
5-Amino-4-chlor-2-phenyl-3(2*H*)-pyridazinon
5-Amino-4-brom-2-phenyl-3(2*H*)-pyridazinon
5-Amino-4-chlor-2-cyclohexyl-3(2*H*)-pyridazinon
5-Amino-4-brom-2-cyclohexyl-3(2*H*)-pyridazinon
5-Methylamino-4-chlor-2-(3-trifluormethylphenyl)-3(2*H*)-pyridazinon
5-Methylamino-4-chlor-2-(3-,α,α,β,β-tetrafluorethoxyphenyl)-3(2*H*)-pyridazinon
5-Dimethylamino-4-chlor-2-phenyl-3(2*H*)-pyridazinon
4,5-Dimethoxy-2-phenyl-3(2*H*)-pyridazinon
4,5-Dimethoxy-2-cyclohexyl-3(2*H*)-pyridazinon
4,5-Dimethoxy-2-(3-trifluormethylphenyl)-3(2*H*)-pyridazinon
5-Methoxy-4-chlor-2-(3-trifluormethylphenyl)-3(2*H*)-pyridazinon
5-Amino-4-brom-2-(3-methylphenyl)-3(2*H*)-pyridazinon
3'-N-Isopropyl-carbamoyloxy-propionsäureanilid
N-3-(4-Fluorphenoxycarbonylamino)-phenylcarbaminsäuremethylester
2-(N-Benzoyl-3,4-dichlorphenylamino)-propionsäureethylester
2-(2-Methyl-4-chlorphenoxy)-N-methoxy-acetamid
α-(3,4,5-Tribompyrazol-1-yl)-N,N-dimethylpropionamid
N-(1,1-Dimethylpropionyl)-3,5-dichlorbenzamid
(4-Bromphenyl)-3,4,5,9,10-pentaazatetracyclo-[5,4,1,$0^{2,6}$,$0,^{8,11}$]-dodeca-3,9-dien
2-Chlorphenoxyessigsäure (Salze, Ester, Amide)
2,4-Dichlorphenyl-3'-ethoxy-ethoxy-ethoxy-4'-nitrophenylether.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mittlen zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

**Patentansprüche**

1. Thiadiazolderivate der Formel

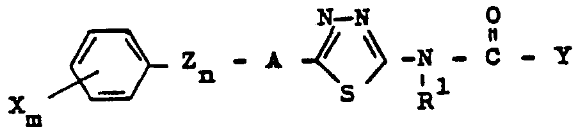

(I),

in der

$R^1$ Wasserstoff oder $C_1$—$C_4$-Alkyl,

A eine gegebenenfalls durch $C_1$—$C_4$-Alkyl substituierte Alkylenkette mit 1 bis 8 Kohlenstoffatomen,

X Wasserstoff, Halogen, Cyano, Nitro, $C_1$—$C_4$-Alkyl, $C_1$—$C_4$-Alkoxy, $C_1$—$C_4$-Halogenalkyl, $C_1$—$C_4$-Halogenalkoxy, $C_1$—$C_4$-Alkylthio, $C_1$—$C_4$-Alkylsulfonyl, $C_3$—$C_6$-Cycloalkyl, Phenyl und gegebenenfalls durch Halogen substituiertes Phenoxy,

m 0, 1, 2, 3 oder 4,

Y $C_1$—$C_4$-Alkyl, $C_3$—$C_6$-Cycloalkyl, $C_1$—$C_4$-Alkoxy, $C_1$—$C_4$-Alkylthio oder den Rest $NR^2R^3$, wobei $R^2$ und $R^3$ unabhängig voneinander Wasserstoff, $C_1$—$C_4$-Alkyl, $C_2$—$C_4$-Alkenyl, $C_2$—$C_4$-Alkinyl, $C_1$—$C_4$-Alkoxy oder $C_3$—$C_6$-Cycloalkyl bedeuten oder $R^2$ mit $R^3$ zusammen eine gegebenenfalls durch Methyl substituierte und eine gegebenenfalls durch Sauerstoff unterbrochene Alkylenkette mit 3 bis 7 Kohlenstoffatomen,

Z Sauerstoff oder Schwefel und

n 0 oder 1 bedeuten und in der anstelle des Restes

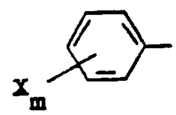

ein gegebenenfalls durch Halogen, $C_1$—$C_4$-Alkyl oder $C_1$—$C_4$-Alkoxy substituierter Naphthylrest stehen kann.

2. Thiadiazolderivate der Formel I gemäß Anspruch 1, dadurch gekennzeichnet, daß $R^1$ $C_1$—$C_4$-Alkyl und Y den Rest $NR^2R^3$, wobei $R^2$ für Wasserstoff und $R^3$ für $C_1$—$C_4$-Alkyl stehen, bedeuten.

3. Thiadiazolderivate der Formel I gemäß Anspruch 1, dadurch gekennzeichnet, daß $R^1$ Methyl und Y den Rest $NR^2R^3$, wobei $R^2$ für Wasserstoff und $R^3$ für Methyl stehen, bedeuten.

4. N-[2-[3-(4'-Chlorphenoxy)-propyl]-1,3,4-thiadiazol-5-yl]-N-methyl-N'-methyl-harnstoff.

5. Herbizid, enthaltend inerte Zusatzstoffe und ein Thiadiazolderivat der Formel

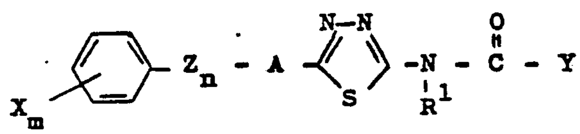

(I),

in der

$R^1$ Wasserstoff oder $C_1$—$C_4$-Alkyl,

A eine gegebenenfalls durch $C_1$—$C_4$-Alkyl substituierte Alkylenkette mit 1 bis 8 Kohlenstoffatomen,

X Wasserstoff, Halogen, Cyano, Nitro, $C_1$—$C_4$-Alkyl, $C_1$—$C_4$-Alkoxy, $C_1$—$C_4$-Halogenalkyl, $C_1$—$C_4$-Halogenalkoxy, $C_1$—$C_4$-Alkylthio, $C_1$—$C_4$-Alkylsulfonyl, $C_3$—$C_6$-Cycloalkyl, Phenyl und gegebenenfalls durch Halogen substituiertes Phenoxy,

m 0, 1, 2, 3 oder 4,

Y $C_1$—$C_4$-Alkyl, $C_3$—$C_6$-Cycloalkyl, $C_1$—$C_4$-Alkoxy, $C_1$—$C_4$-Alkylthio oder den Rest $NR^2R^3$, wobei $R^2$ und $R^3$ unabhängig voneinander Wasserstoff, $C_1$—$C_4$-Alkyl, $C_2$—$C_4$-Alkenyl, $C_2$—$C_4$-Alkinyl, $C_1$—$C_4$-Alkoxy oder $C_3$—$C_6$-Cycloalkyl bedeuten oder $R^2$ mit $R^3$ zusammen eine gegebenenfalls durch Methyl substituierte und eine gegebenenfalls durch Sauerstoff unterbrochene Alkylenkette mit 3 bis 7 Kohlenstoffatomen,

Z Sauerstoff oder Schwefel und

n 0 oder 1 bedeuten und in der anstelle des Restes

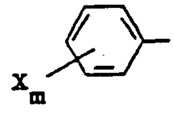

ein gegebenenfalls durch Halogen, $C_1$—$C_4$-Alkyl oder $C_1$—$C_4$-Alkoxy substituierter Naphthylrest stehen kann.

6. Verfahren zur Herstellung der Thiadiazolderivate der Formel I gemäß Anspruch 1, in der $R^1$, A, X, Z, m und n die im Anspruch 1 genannten Bedeutungen haben und Y den Rest $NR^2R^3$ bedeutet, wobei $R^2$ für Wasserstoff steht und $R^3$ die im Anspruch 1 genannten Bedeutungen hat, dadurch gekennzeichnet, daß man ein Aminothiadiazol der Formel

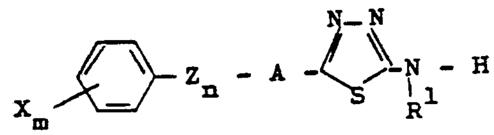

(II),

in der

$R^1$, A, X, Z, m und n die im Anspruch 1 genannten Bedeutungen haben, gegebenenfalls in Gegenwart eines unter den Reaktionsbedingungen inerten Lösungsmittels bei einer Temperatur zwischen −20 und +150°C mit einem Isocyanat der Formel

$R^3$—NCO (IV),

in der $R^3$ die im Anspruch 1 genannten Bedeutungen hat, umsetzt.

7. Verfahren zur Herstellung der Thiadiazolderivate der Formel I gemäß Anspruch 1, dadurch gekennzeichnet, daß man ein Aminothiadiazol der Formel

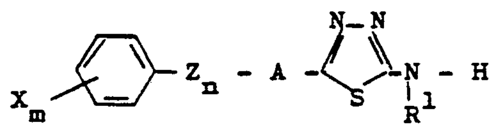

(II),

in der

$R^1$, A, X, Z, m und n die im Anspruch 1 genannten Bedeutungen haben, gegebenenfalls in Gegenwart eines säurebindenden Mittles und in Gegenwart eines unter den Reaktionsbedingungen inerten Lösungsmittels bei einer Temperatur zwischen −20 und +150°C mit einem Säurechlorid der Formel

Y—CO—Hal (III),

in der

Y die im Anspruch 1 genannten Bedeutungen hat und Hal Halogen bedeutet,

umsetzt.

8. Verfahren zur Bekämpfung unerwünschten Pflanzenwuches, dadurch gekennzeichnet, daß man ein Thiadiazolderivat der Formel I gemäß Anspruch 1 auf die Pflanzen und/oder ihren Standort einwirken läßt.

**Claims**

1. A thiadiazole derivative of the formula

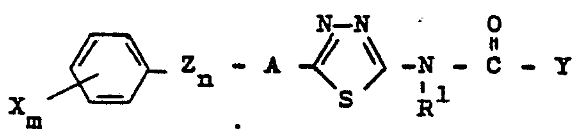

(I),

where

$R^1$ is hydrogen or $C_1$—$C_4$-alkyl,

A is an alkylene chain of 1 to 8 carbon atoms which is unsubstituted or substituted by $C_1$—$C_4$-alkyl,

X is hydrogen, halogen, cyano, nitro, $C_1$—$C_4$-alkyl, $C_1$—$C_4$-alkoxy, $C_1$—$C_4$-haloalkyl, $C_1$—$C_4$-haloalkoxy, $C_1$—$C_4$-alkylthio, $C_1$—$C_4$-alkylsulfonyl, $C_3$—$C_6$-cycloalkyl, phenyl, or unsubstituted or halogen-substituted phenoxy,

m is 0, 1, 2, 3 or 4,

Y is $C_1$—$C_4$-alkyl, $C_3$—$C_6$-cycloalkyl, $C_1$—$C_4$-alkoxy, $C_1$—$C_4$-alkylthio or $NR^2R^3$, where $R^2$ and $R^3$ independently of one another are hydrogen, $C_1$—$C_4$-alkyl, $C_2$—$C_4$-alkenyl, $C_2$—$C_4$-alkynyl, $C_1$—$C_4$-alkoxy or $C_3$—$C_6$-cycloalkyl, or $R^2$ and $R^3$ together form an alkylene chain of 3 to 7 carbon atoms which is unsubstituted or methyl-substituted and may or may not be interrupted by oxygen,

Z is oxygen or sulfur, and
n is 0 or 1, and where the radical

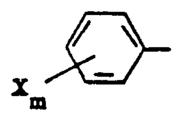

may be replaced by naphthyl which is unsubstituted or substituted by halogen, $C_1$—$C_4$-alkyl or $C_1$—$C_4$-alkoxy.

2. A thiadiazole derivative of the formula I as claimed in claim 1, where $R^1$ is $C_1$—$C_4$-alkyl and Y is $NR^2R^3$, $R^2$ denoting hydrogen and $R^3$ $C_1$—$C_4$-alkyl.

3. A thiadiazole derivative of the formula I as claimed in claim 1, where $R^1$ is methyl and Y is $NR^2R^3$, $R^2$ denoting hydrogen and $R^3$ methyl.

4. N-[2-[3-(4′-Chlorphenoxy)-propyl]-1,3,4-thiadiazol-5-yl]-N-methyl-N′-methylurea.

5. A herbicide containing inert additives and a thiadiazole derivative of the formula

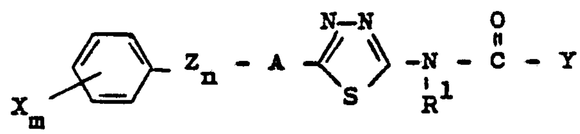 (I),

where
$R^1$ is hydrogen or $C_1$—$C_4$-alkyl,
A is an alkylene chain of 1 to 8 carbon atoms which is unsubstituted or substituted by $C_1$—$C_4$-alkyl,
X is hydrogen, halogen, cyano, nitro, $C_1$—$C_4$-alkyl, $C_1$—$C_4$-alkoxy, $C_1$—$C_4$-haloalkyl, $C_1$—$C_4$-haloalkoxy, $C_1$—$C_4$-alkylthio, $C_1$—$C_4$-alkylsulfonyl, $C_3$—$C_8$-cycloalkyl, phenyl, or unsubstituted or halogen-substituted phenoxy,
m is 0, 1, 2, 3 or 4,
Y is $C_1$—$C_4$-alkyl, $C_3$—$C_6$-cycloalkyl, $C_1$—$C_4$-alkoxy, $C_1$—$C_4$-alkylthio or $NR^2R^3$, where $R^2$ and $R^3$ independently of one another are hydrogen, $C_1$—$C_4$-alkyl, $C_2$—$C_4$-alkenyl, $C_2$—$C_4$-alkynyl, $C_1$—$C_4$-alkoxy or $C_3$—$C_6$-cycloalkyl, or $R^2$ and $R^3$ together form an alkylene chain of 3 to 7 carbon atoms which is unsubstituted or methyl-substituted and may or may not be interrupted by oxygen,
Z is oxygen or sulfur, and
n is 0 or 1, and where the radical

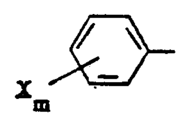

may be replaced by naphthyl which is unsubstituted or substituted by halogen, $C_1$—$C_4$-alkyl or $C_1$—$C_4$-alkoxy.

6. A process for the preparation of a thiadiazole derivative of the formula I as claimed in claim 1, where $R^1$, A, X, Z, m and n have the meanings given in claim 1, and Y is $NR^2R^3$, $R^2$ denoting hydrogen and $R^3$ having the meanings given in claim 1, wherein an aminothiadiazole of the formula

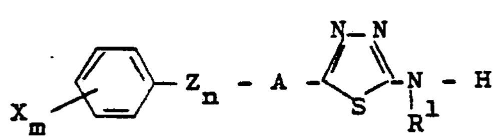 (II),

where
$R^1$, A, X, Z, m and n have the meanings given in claim 1, is reacted with an isocyanate of the formula

$$R^3—NCO \quad (IV)$$

where
$R^3$ has the meanings given in claim 1, at a temperature of from −20 to +150°C, in the presence or absence of a solvent which is inert under the reaction conditions.

7. A process for the preparation of a thiadiazole derivative of the formula I as claimed in claim 1, wherein an aminothiadiazole of the formula

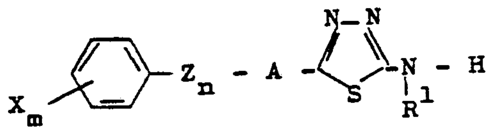 (II),

where

$R^1$, A, X, Z, m and n have the meanings given in claim 1, is reacted with an acid chloride of the formula

Y—CO—Hal (III),

where

Y has the meanings given in claim 1 and Hal is halogen, at a temperature of from −20 to +150°C, in the presence or absence of an acid acceptor and a solvent which is inert under the reaction conditions.

8. A process for combating unwanted plant growth, wherein a thiadiazole derivative of the formula I as claimed in claim 1 is allowed to act on the plants and/or their location.

**Revendications**

1. Dérivés de thiadiazole de formule

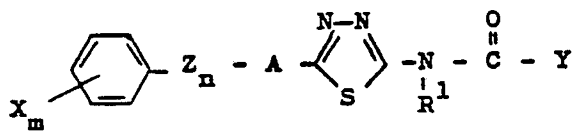 (I),

dans laquelle

$R^1$ représente hydrogène ou alkyle en $C_1$—$C_4$

A une chaîne alkylène de 1 à 8 atomes de carbone, éventuellement substituée par alkyle en $C_1$—$C_4$

X hydrogène, halogène, cyano, nitro, alkyle en $C_1$—$C_4$, alcoxy en $C_1$—$C_4$, halogénalkyle en $C_1$—$C_4$, halogénalcoxy en $C_1$—$C_4$, alkylthio en $C_1$—$C_4$, alkylsulfonyle en $C_1$—$C_4$, cycloalkyle en $C_3$—$C_6$, phényle et phénoxy, éventuellement substitué par halogène,

m est 0, 1, 2, 3 ou 4

Y représente alkyle en $C_1$—$C_4$, cycloalkyle en $C_3$—$C_6$, alcoxy en $C_1$—$C_4$, alkylthio en $C_1$—$C_4$ ou le reste $NR^2R^3$, $R^2$ et $R^3$ représentant, indépendamment l'une de l'autre, hydrogène, alkyle en $C_1$—$C_4$, alcényle en $C_2$—$C_4$, alcinyle en $C_2$—$C_4$, alcoxy en $C_1$—$C_4$ ou cycloalkyle en $C_3$—$C_6$, ou bien $R^2$ avec $R^3$ représentant ensemble une chaîne alkylène de 3 à 7 atomes de carbone, éventuellement substituée par méthyle et une telle chaîne éventuellement interrompue par oxygène,

Z représente oxygène ou soufre et

n représente 0 ou 1 et dans laquelle, à la place du reste

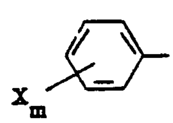,

peut se trouver un reste naphtyle éventuellement substitué par halogène, alkyle en $C_1$—$C_4$ ou alcoxy en $C_1$—$C_4$.

2. Dérivés de thiadiazole de formule I selon la revendication 1, caractérisés par le fait que $R^1$ représente alkyle en $C_1$—$C_4$ et Y le reste $NR^2R^3$, $R^2$ étant mis pour hydrogène et $R^3$ pour alkyle en $C_1$—$C_4$.

3. Dérivés de thiadiazole de formule I selon la revendication 1, caractérisés par le fait que $R^1$ représente méthyle et Y le reste $NR^2R^3$, $R^2$ étant mis pour hydrogène et $R^3$ pour méthyle

4. N-[2-[3-(4'-chlorophénoxy)-propyl]-1,3,4-thiadiazol-5-yl]-N-méthyl-N'-méthyl-urée.

5. Herbicide contenant des additifs inertes et un dérivé de thiadiazole de formule

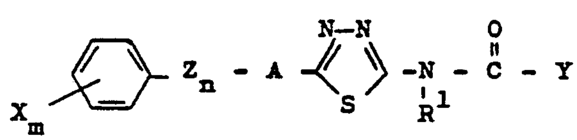 (I),

dans laquelle

$R^1$ représente hydrogène ou alkyle en $C_1$—$C_4$

A une chaîne alkylène de 1 à 8 atomes de carbone, éventuellement substituée par alkyle en $C_1$—$C_4$

X hydrogène, halogène, cyano, nitro, alkyle en $C_1$—$C_4$, alcoxy en $C_1$—$C_4$, halogénalkyle en $C_1$—$C_4$, halogénalcoxy en $C_1$—$C_4$, alkylthio en $C_1$—$C_4$, alkylsulfonyle en $C_1$—$C_4$, cycloalkyle en $C_3$—$C_6$, phényle et phénoxy, éventuellement substitué par halogène,

m est 0, 1, 2, 3 ou 4

Y représente alkyle en $C_1$—$C_4$, cycloalkyle en $C_3$—$C_6$, alcoxy en $C_1$—$C_4$, alkylthio en $C_1$—$C_4$ ou le reste $NR^2R^3$, $R^2$ et $R^3$ représentant, indépendamment l'une de l'autre, hydrogène, alkyle en $C_1$—$C_4$, alcényle en $C_2$—$C_4$, alcinyle en $C_2$—$C_4$, alcoxy en $C_1$—$C_4$ ou cycloalkyle en $C_3$—$C_6$, ou bien $R^2$ avec $R^3$ représentant ensemble une chaîne alkylène de 3 à 7 atomes de carbone, éventuellement substituée par méthyle et une telle chaîne éventuellement interrompue par oxygène,

Z représente oxygène ou soufre et

n représente 0 ou 1, et dans laquelle, à place du reste

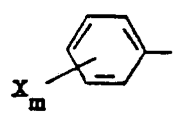,

peut se trouver un reste naphtyle éventuellement substitué par halogène, alkyle en $C_1$—$C_4$ ou alcoxy en $C_1$—$C_4$.

6. Procédé de préparation de dérivés de thiadiazole de formule I selon la revendication 1, dans laquelle $R^1$, A, X, Z, m et n ont les significations données dans la revendication 1 et Y représente le reste $NR^2R^3$, $R^2$ étant mis pour hydrogène et $R^3$ ayant la signification donnée dans la revendication 1, caractérisé par le fait que l'on fait réagir un aminothiadiazole de formule

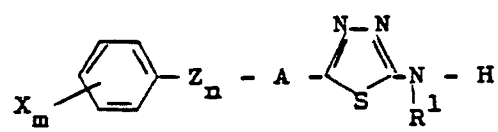

(II),

dans laquelle, $R^1$, A, X, Z, m et n ont les significations données dans la revendication 1, éventuellement en présence d'un solvant inerte dans les conditions de réaction, à une température comprise entre −20 et +150°C, avec un isocyanate de formule

$$R^3—NCO \qquad (IV)$$

dans laquelle $R^3$ a la signification donnée dans le revendication 1.

7. Procédé de préparation de dérivés de thiadiazole de formule I selon la revendication 1, caractérisé par le fait que l'on fait réagir un aminothiadiazole de formule

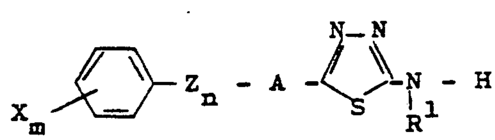

(II),

dans laquelle, $R^1$, A, X, Z, m et n ont les significations données dans la revendication 1, éventuellement en présence d'un agent liant les acides et en présence d'un solvant inerte dans les conditions de réaction, à une température comprise entre −20 et +150°C, avec un chlorure d'acide de formule

$$Y—CO—Hal \qquad (III),$$

dans laquelle Y a la signification donnée dans la revendication 1 et Hal représente halogène.

8. Procédé de lutte contre la croissance de plantes indésirables, caractérisé par le fait que l'on fait agir un dérivé de thiadiazole de formule 1 selon la revendication 1 sur les plantes et/ou leur biotope.